# EUROPEAN PATENT SPECIFICATION

(11) **EP 4 380 489 B1**
(45) Date of publication and mention of the grant of the patent: **16.04.2025**
(21) Application number: 22754172.9
(22) Date of filing: 26.07.2022
(51) Int. Cl.: A61B 34/30, A61B 90/00, A61B 34/37

(54) **SYSTEM AND METHOD FOR SURGICAL INSTRUMENT USE PREDICTION**
SYSTEM UND VERFAHREN ZUR VORHERSAGE DER VERWENDUNG EINES CHIRURGISCHEN INSTRUMENTS
SYSTÈME ET PROCÉDÉ DE PRÉDICTION D'UTILISATION D'INSTRUMENT CHIRURGICAL

(30) Priority: 03.08.2021 US 202163228656 P
(43) Date of publication of application: 12.06.2024
(73) Proprietor: Covidien LP, Mansfield, MA 02048 (US)
(72) Inventor: ROCKROHR, Brian A., Minneapolis, Minnesota 55432 (US)
(74) Representative: Maschio & Soames IP Ltd
(86) International application number: PCT/IB2022/056857
(87) International publication number: WO 2023/012574

(56) References cited:
- US-A1- 2019 206 565
- US-A1- 2020 345 437
- US-B1- 9 247 996

## Description

### BACKGROUND

### Technical Field

The present disclosure generally relates to surgical instruments and in particular, to tracking use of the surgical instrument and predicting a number of remaining uses based on prior use patterns.

### Background of Related Art

Surgical robotic systems are currently being used in minimally invasive medical procedures. Some surgical robotic systems include a surgeon console controlling a surgical robotic arm and a surgical instrument having an end effector (e.g., forceps or grasping instrument) coupled to and actuated by the robotic arm. In operation, the robotic arm is moved to a position over a patient and then guides the surgical instrument into a small incision via a surgical port or a natural orifice of a patient to position the end effector at a work site within the patient's body.

Surgical instruments used with robotic systems have a finite life due to wear and tear imparted on surgical instruments' components. Thus, there is a need for a system and a method to track and predict instrument use to prevent using the surgical instruments beyond their preset lifetimes. Background prior art is disclosed in US 2020/345437 A1, US 2019/206565 A1 and US 9 247 996 B1.

### SUMMARY

The invention is defined by independent claims 1, 8.

According to one aspect of the present disclosure, a surgical robotic system is disclosed, which includes a robotic arm; a surgical instrument configured to couple to the robotic arm, and a controller configured to: access usage data pertaining the surgical instrument; calculate a predicted number of uses remaining for the surgical instrument; and disable the surgical instrument based on the predicted number of uses.

Implementations of the above embodiment may include one or more of the following features. According to one aspect of the above embodiment, the usage data may include maximum life data and previous use data. The maximum life data may include a number of maximum uses and a maximum use time. The previous use data may include a number of previous uses, a previous use time, and an expired flag. The controller may be further configured to disable the surgical instrument based on the expired flag being set to true. The controller may be further configured to disable the surgical instrument based on the previous use time exceeding the maximum use time. The controller may be further configured to disable the surgical instrument based on the number of previous uses exceeding the number of maximum uses. The controller may be further configured to calculate an average use time based on the previous use time and the number of previous uses. The controller may be further configured to calculate the predicted number of uses based on the maximum use time and the average use time. The surgical robotic system may further include a display configured to output a graphical user interface may including a usage indicator status pertaining the surgical instrument. The surgical instrument may also include a storage device configured to store the usage data. The controller may be further configured to update the usage data following the use of the surgical instrument.

According to another aspect of the present disclosure, a method for controlling a surgical system is disclosed and includes accessing usage data pertaining to a surgical instrument; calculating a predicted number of uses remaining for the surgical instrument, and disabling the surgical instrument based on the predicted number of uses.

Implementations of the above embodiment may include one or more of the following features. According to one aspect of the above embodiment, accessing the usage data may include accessing maximum life data and previous use data. Accessing the maximum life data may include accessing a number of maximum uses and a maximum use time. Accessing the previous use data may include accessing a number of previous uses, a previous use time, and an expired flag. Calculating the predicted number of uses further may include calculating an average use time based on the previous use time and the number of previous uses. Calculating the predicted number of uses is based on the maximum use time and the average use time. The method may also include displaying on a graphical user interface a usage indicator status pertaining the surgical instrument. The method may also include updating the usage data following the use of the surgical instrument.

### BRIEF DESCRIPTION OF THE DRAWINGS

Various aspects of the present disclosure are described herein with reference to the drawings wherein:
FIG. 1 is a schematic illustration of a surgical robotic system including a control tower, a console, and one or more surgical robotic arms each disposed on a movable cart according to an aspect of the present disclosure;
FIG. 2 is a perspective view of a surgical robotic arm of the surgical robotic system of FIG. 1 according to an aspect of the present disclosure;
FIG. 3 is a perspective view of a setup arm with the surgical robotic arm of the surgical robotic system of FIG. 1 according to an aspect of the present disclosure;
FIG. 4 is a schematic diagram of a computer architecture of the surgical robotic system of FIG. 1 according to an aspect of the present disclosure;
FIG. 5 is a rear perspective view of a surgical instrument;
FIG. 6 is a graphical user interface displayed on a display of the surgical robotic system of FIG. 1 according to an embodiment of the present disclosure; and
FIG. 7 is a flow chart of a method for instrument use prediction according to the present disclosure.

### DETAILED DESCRIPTION

The term "application" may include a computer program designed to perform functions, tasks, or activities for the benefit of a user. Application may refer to, for example, software running locally or remotely, as a standalone program or in a web browser, or other software which would be understood by one skilled in the art to be an application. An application may run on a controller, or on a user device, including, for example, a mobile device, a personal computer, or a server system.

As will be described in detail below, the present disclosure is directed to a surgical robotic system, which includes a surgeon console, a control tower, and one or more movable carts having a surgical robotic arm coupled to a setup arm. The surgeon console receives user input through one or more interface devices, which are interpreted by the control tower as movement commands for moving the surgical robotic arm. The surgical robotic arm includes a controller, which is configured to process the movement command and to generate a torque command for activating one or more actuators of the robotic arm, which would, in turn, move the robotic arm in response to the movement command.

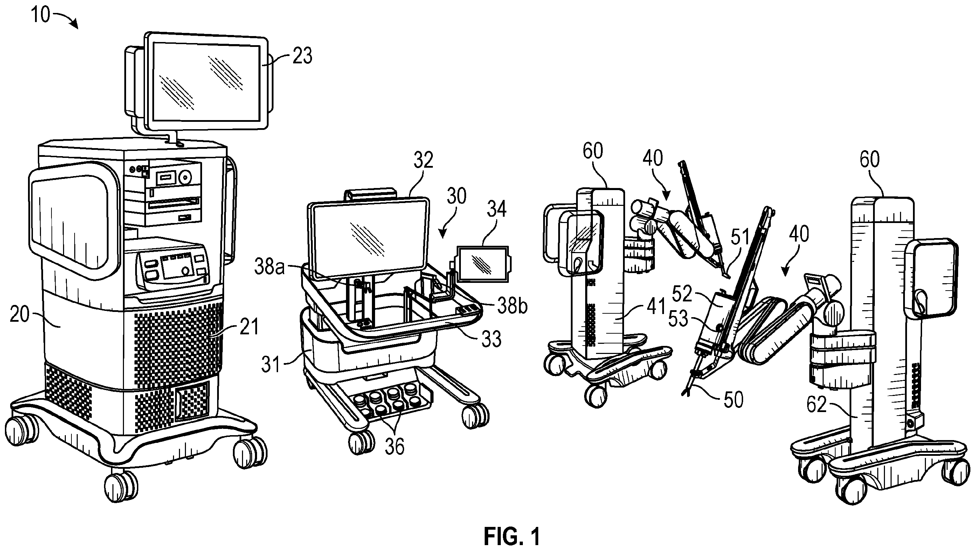

With reference to FIG. 1, a surgical robotic system 10 includes a control tower 20, which is connected to all of the components of the surgical robotic system 10 including a surgeon console 30 and one or more robotic arms 40. Each of the robotic arms 40 includes a surgical instrument 50 removably coupled thereto. Each of the robotic arms 40 is also coupled to a movable cart 60.

The surgical instrument 50 is configured for use during minimally invasive surgical procedures. In aspects, the surgical instrument 50 may be configured for open surgical procedures. In aspects, the surgical instrument 50 may be an endoscope, such as an endoscopic camera 51, configured to provide a video feed for the user. In further aspects, the surgical instrument 50 may be an electrosurgical forceps configured to seal tissue by compressing tissue between jaw members and applying electrosurgical current thereto. In yet further aspects, the surgical instrument 50 may be a surgical stapler including a pair of jaws configured to grasp and clamp tissue while deploying a plurality of tissue fasteners, e.g., staples, and cutting stapled tissue.

One of the robotic arms 40 may include the endoscopic camera 51 configured to capture video of the surgical site. The endoscopic camera 51 may be a stereoscopic endoscope configured to capture two side-by-side (i.e., left and right) images of the surgical site to produce a video stream of the surgical scene. The endoscopic camera 51 is coupled to a video processing device 56, which may be disposed within the control tower 20. The video processing device 56 may be any computing device as described below configured to receive the video feed from the endoscopic camera 51 perform the image processing based on the depth estimating algorithms of the present disclosure and output the processed video stream.

The surgeon console 30 includes a first display 32, which displays a video feed of the surgical site provided by camera 51 of the surgical instrument 50 disposed on the robotic arms 40, and a second display 34, which displays a user interface for controlling the surgical robotic system 10. The first and second displays 32 and 34 are touchscreens allowing for displaying various graphical user inputs. The video processing device 56 is configured to process the video feed from the endoscopic camera 51 and to output a processed video stream on the first displays 32 of the surgeon console 30 and/or the display 23 of the control tower 20.

The surgeon console 30 also includes a plurality of user interface devices, such as foot pedals 36 and a pair of handle controllers 38a and 38b which are used by a user to remotely control robotic arms 40. The surgeon console further includes an armrest 33 used to support clinician's arms while operating the handle controllers 38a and 38b.

The control tower 20 includes a display 23, which may be a touchscreen, and outputs on the graphical user interfaces (GUIs). The control tower 20 also acts as an interface between the surgeon console 30 and one or more robotic arms 40. In particular, the control tower 20 is configured to control the robotic arms 40, such as to move the robotic arms 40 and the corresponding surgical instrument 50, based on a set of programmable instructions and/or input commands from the surgeon console 30, in such a way that robotic arms 40 and the surgical instrument 50 execute a desired movement sequence in response to input from the foot pedals 36 and the handle controllers 38a and 38b.

Each of the control tower 20, the surgeon console 30, and the robotic arm 40 includes a respective computer 21, 31, 41. The computers 21, 31, 41 are interconnected to each other using any suitable communication network based on wired or wireless communication protocols. The term "network," whether plural or singular, as used herein, denotes a data network, including, but not limited to, the Internet, Intranet, a wide area network, or a local area networks, and without limitation as to the full scope of the definition of communication networks as encompassed by the present disclosure. Suitable protocols include, but are not limited to, transmission control protocol/internet protocol (TCP/IP), datagram protocol/internet protocol (UDP/IP), and/or datagram congestion control protocol (DCCP). Wireless communication may be achieved via one or more wireless configurations, e.g., radio frequency, optical, Wi-Fi, Bluetooth (an open wireless protocol for exchanging data over short distances, using short length radio waves, from fixed and mobile devices, creating personal area networks (PANs), ZigBee^{®} (a specification for a suite of high level communication protocols using small, low-power digital radios based on the IEEE 122.15.4-2003 standard for wireless personal area networks (WPANs)).

The computers 21, 31, 41 may include any suitable processor (not shown) operably connected to a memory (not shown), which may include one or more of volatile, non-volatile, magnetic, optical, or electrical media, such as read-only memory (ROM), random access memory (RAM), electrically-erasable programmable ROM (EEPROM), non-volatile RAM (NVRAM), or flash memory. The processor may be any suitable processor (e.g., control circuit) adapted to perform the operations, calculations, and/or set of instructions described in the present disclosure including, but not limited to, a hardware processor, a field programmable gate array (FPGA), a digital signal processor (DSP), a central processing unit (CPU), a microprocessor, and combinations thereof. Those skilled in the art will appreciate that the processor may be substituted for by using any logic processor (e.g., control circuit) adapted to execute algorithms, calculations, and/or set of instructions described herein.

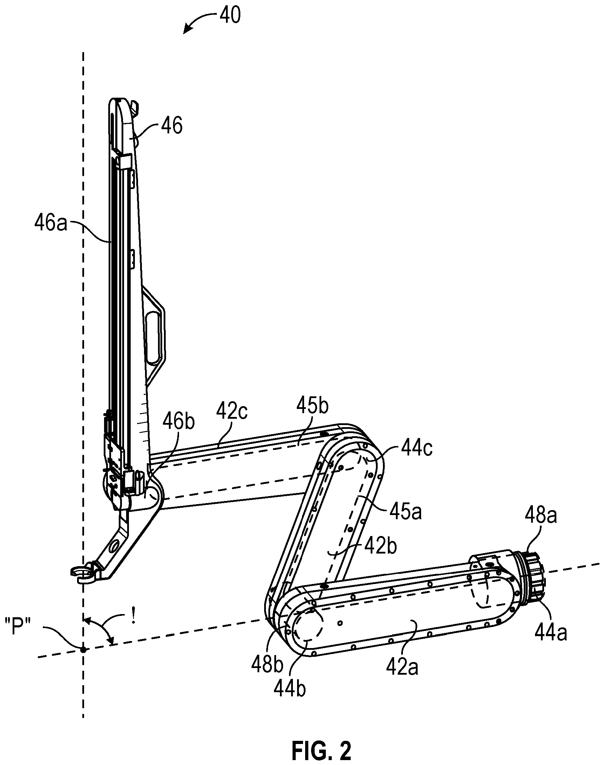

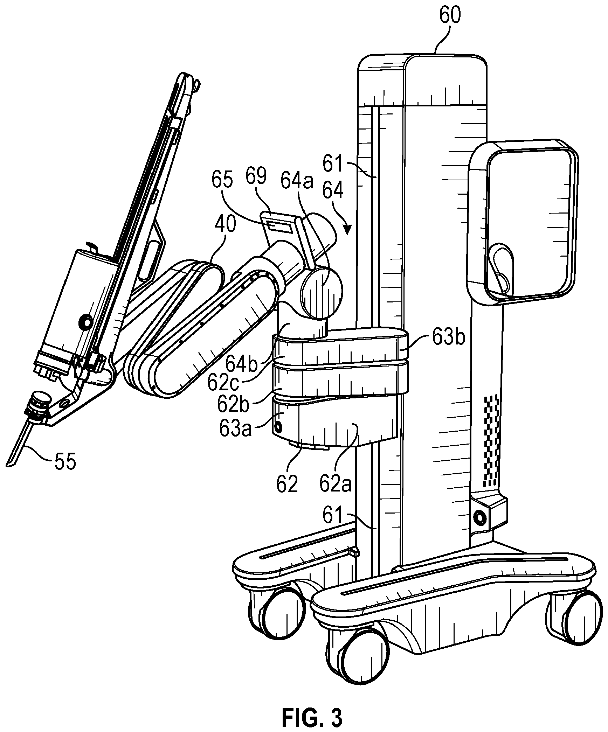

With reference to FIG. 2, each of the robotic arms 40 may include a plurality of links 42a, 42b, 42c, which are interconnected at joints 44a, 44b, 44c, respectively. The joint 44a is configured to secure the robotic arm 40 to the movable cart 60 and defines a first longitudinal axis. With reference to FIG. 3, the movable cart 60 includes a lift 61 and a setup arm 62, which provides a base for mounting of the robotic arm 40. The lift 61 allows for vertical movement of the setup arm 62. The movable cart 60 also includes a display 69 for displaying information pertaining to the robotic arm 40.

The setup arm 62 includes a first link 62a, a second link 62b, and a third link 62c, which provide for lateral maneuverability of the robotic arm 40. The links 62a, 62b, 62c are interconnected at joints 63a and 63b, each of which may include an actuator (not shown) for rotating the links 62b and 62b relative to each other and the link 62c. In particular, the links 62a, 62b, 62c are movable in their corresponding lateral planes that are parallel to each other, thereby allowing for extension of the robotic arm 40 relative to the patient (e.g., surgical table). In aspects, the robotic arm 40 may be coupled to a surgical table. The setup arm 62 includes controls 65 for adjusting movement of the links 62a, 62b, 62c as well as the lift 61.

The third link 62c includes a rotatable base 64 having two degrees of freedom. In particular, the rotatable base 64 includes a first actuator 64a and a second actuator 64b. The first actuator 64a is rotatable about a first stationary arm axis which is perpendicular to a plane defined by the third link 62c and the second actuator 64b is rotatable about a second stationary arm axis which is transverse to the first stationary arm axis. The first and second actuators 64a and 64b allow for full three-dimensional orientation of the robotic arm 40.

The actuator 48b of the joint 44b is coupled to the joint 44c via the belt 45a, and the joint 44c is in turn coupled to the joint 46c via the belt 45b. Joint 44c may include a transfer case coupling the belts 45a and 45b, such that the actuator 48b is configured to rotate each of the links 42b, 42c and the holder 46 relative to each other. More specifically, links 42b, 42c, and the holder 46 are passively coupled to the actuator 48b which enforces rotation about a pivot point "P" which lies at an intersection of the first axis defined by the link 42a and the second axis defined by the holder 46. Thus, the actuator 48b controls the angle θ between the first and second axes allowing for orientation of the surgical instrument 50. Due to the interlinking of the links 42a, 42b, 42c, and the holder 46 via the belts 45a and 45b, the angles between the links 42a, 42b, 42c, and the holder 46 are also adjusted in order to achieve the desired angle θ. In aspects, some or all of the joints 44a, 44b, 44c may include an actuator to obviate the need for mechanical linkages.

The joints 44a and 44b include an actuator 48a and 48b configured to drive the joints 44a, 44b, 44c relative to each other through a series of belts 45a and 45b or other mechanical linkages such as a drive rod, a cable, or a lever and the like. In particular, the actuator 48a is configured to rotate the robotic arm 40 about a longitudinal axis defined by the link 42a.

With reference to FIG. 2, the robotic arm 40 also includes a holder 46 defining a second longitudinal axis and configured to receive an instrument drive unit (IDU) 52 (FIG. 1). The IDU 52 is configured to couple to an actuation mechanism of the surgical instrument 50 and the camera 51 and is configured to move (e.g., rotate) and actuate the instrument 50 and/or the camera 51. IDU 52 transfers actuation forces from its actuators to the surgical instrument 50 to actuate components (e.g., end effector) of the surgical instrument 50. The holder 46 includes a sliding mechanism 46a, which is configured to move the IDU 52 along the second longitudinal axis defined by the holder 46. The holder 46 also includes a joint 46b, which rotates the holder 46 relative to the link 42c. During endoscopic procedures, the instrument 50 may be inserted through an endoscopic port 55 (FIG. 3) held by the holder 46.

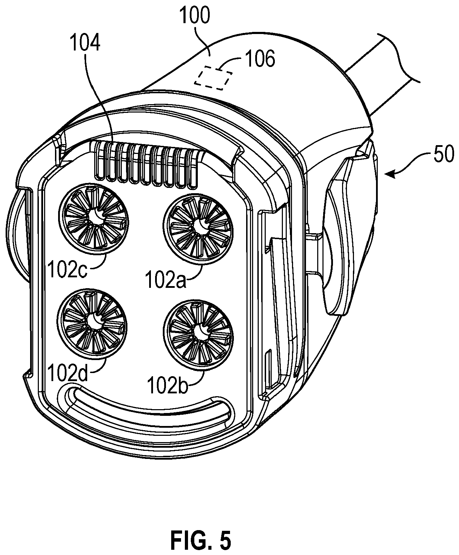

The robotic arm 40 also includes a plurality of manual override buttons 53 (FIGS. 1 and 5) disposed on the IDU 52 and the setup arm 62, which may be used in a manual mode. The user may press one or more of the buttons 53 to move the component associated with the button 53.

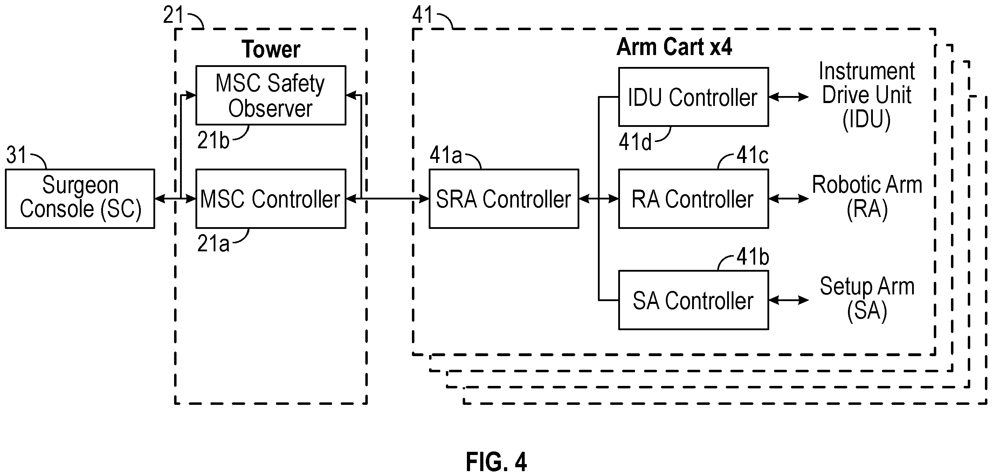

With reference to FIG. 4, each of the computers 21, 31, 41 of the surgical robotic system 10 may include a plurality of controllers, which may be embodied in hardware and/or software. The computer 21 of the control tower 20 includes a controller 21a and safety observer 21b. The controller 21a receives data from the computer 31 of the surgeon console 30 about the current position and/or orientation of the handle controllers 38a and 38b and the state of the foot pedals 36 and other buttons. The controller 21a processes these input positions to determine desired drive commands for each joint of the robotic arm 40 and/or the IDU 52 and communicates these to the computer 41 of the robotic arm 40. The controller 21a also receives the actual joint angles measured by encoders of the actuators 48a and 48b and uses this information to determine force feedback commands that are transmitted back to the computer 31 of the surgeon console 30 to provide haptic feedback through the handle controllers 38a and 38b. The safety observer 21b performs validity checks on the data going into and out of the controller 21a and notifies a system fault handler if errors in the data transmission are detected to place the computer 21 and/or the surgical robotic system 10 into a safe state.

The computer 41 includes a plurality of controllers, namely, a main cart controller 41a, a setup arm controller 41b, a robotic arm controller 41c, and an instrument drive unit (IDU) controller 41d. The main cart controller 41a receives and processes joint commands from the controller 21a of the computer 21 and communicates them to the setup arm controller 41b, the robotic arm controller 41c, and the IDU controller 41d. The main cart controller 41a also manages instrument exchanges and the overall state of the movable cart 60, the robotic arm 40, and the IDU 52. The main cart controller 41a also communicates actual joint angles back to the controller 21a.

The setup arm controller 41b controls each of joints 63a and 63b, and the rotatable base 64 of the setup arm 62 and calculates desired motor movement commands (e.g., motor torque) for the pitch axis and controls the brakes. The robotic arm controller 41c controls each joint 44a and 44b of the robotic arm 40 and calculates desired motor torques required for gravity compensation, friction compensation, and closed loop position control of the robotic arm 40. The robotic arm controller 41c calculates a movement command based on the calculated torque. The calculated motor commands are then communicated to one or more of the actuators 48a and 48b in the robotic arm 40. The actual joint positions are then transmitted by the actuators 48a and 48b back to the robotic arm controller 41c.

The IDU controller 41d receives desired joint angles for the surgical instrument 50, such as wrist and jaw angles, and computes desired currents for the motors in the IDU 52. The IDU controller 41d calculates actual angles based on the motor positions and transmits the actual angles back to the main cart controller 41a.

The robotic arm 40 is controlled in response to a pose of the handle controller controlling the robotic arm 40, e.g., the handle controller 38a, which is transformed into a desired pose of the robotic arm 40 through a hand eye transform function executed by the controller 21a. The hand eye function, as well as other functions described herein, is/are embodied in software executable by the controller 21a or any other suitable controller described herein. The pose of one of the handle controller 38a may be embodied as a coordinate position and role-pitch-yaw ("RPY") orientation relative to a coordinate reference frame, which is fixed to the surgeon console 30. The desired pose of the instrument 50 is relative to a fixed frame on the robotic arm 40. The pose of the handle controller 38a is then scaled by a scaling function executed by the controller 21a. In aspects, the coordinate position is scaled down and the orientation is scaled up by the scaling function. In addition, the controller 21a also executes a clutching function, which disengages the handle controller 38a from the robotic arm 40. In particular, the controller 21a stops transmitting movement commands from the handle controller 38a to the robotic arm 40 if certain movement limits or other thresholds are exceeded and in essence acts like a virtual clutch mechanism, e.g., limits mechanical input from effecting mechanical output.

The desired pose of the robotic arm 40 is based on the pose of the handle controller 38a and is then passed by an inverse kinematics function executed by the controller 21a. The inverse kinematics function calculates angles for the joints 44a, 44b, 44c of the robotic arm 40 that achieve the scaled and adjusted pose input by the handle controller 38a. The calculated angles are then passed to the robotic arm controller 41c, which includes a joint axis controller having a proportional-derivative (PD) controller, the friction estimator module, the gravity compensator module, and a two-sided saturation block, which is configured to limit the commanded torque of the motors of the joints 44a, 44b, 44c.

With reference to FIG. 5, the instrument 50 includes a housing assembly 100 enclosing drive couplers 102a-d configured for selective connection to the IDU 52. The IDU 52 includes a plurality of motors configured to actuate each of the drive couplers 102a-d thereby actuating the instrument 50. The housing assembly 100 also includes an electrical connector 104 configured for selective connection to the IDU 52. The instrument 50 may include electronics, including, and not limited to, a memory 106, wired or wireless communication circuitry for receiving and transmitting data or information. The IDU 52 may be configured to permit passage or routing of a dedicated electrocautery cable or the like for use and connection to an electrosurgical based electromechanical surgical instrument (e.g., for ablation, coagulation, sealing, etc.).

The memory 106 may be any suitable storage device, such as flash memory and is configured to store identification information of the instrument 50, usage data, and the like. The memory 106 may be accessed by any controllers of the surgical robotic system 10, which may be the computers 21, 31, 41. In an exemplary embodiment, the main controller 21a is configured to read and write to the memory 106 including retrieving and updating usage data. The computers 21, 31, 41 are configured to communicate with the memory 106 through the electrical connector 104 and/or any other wired or wireless interface.

Usage data may be stored in any suitable data structure and includes actual time used, which may be measured in seconds, minutes, etc., number of uses. The main controller 21a uses the usage data to calculate remaining life of the instrument 50 and displays the remaining life on any of the displays 23, 32, and/or 34.

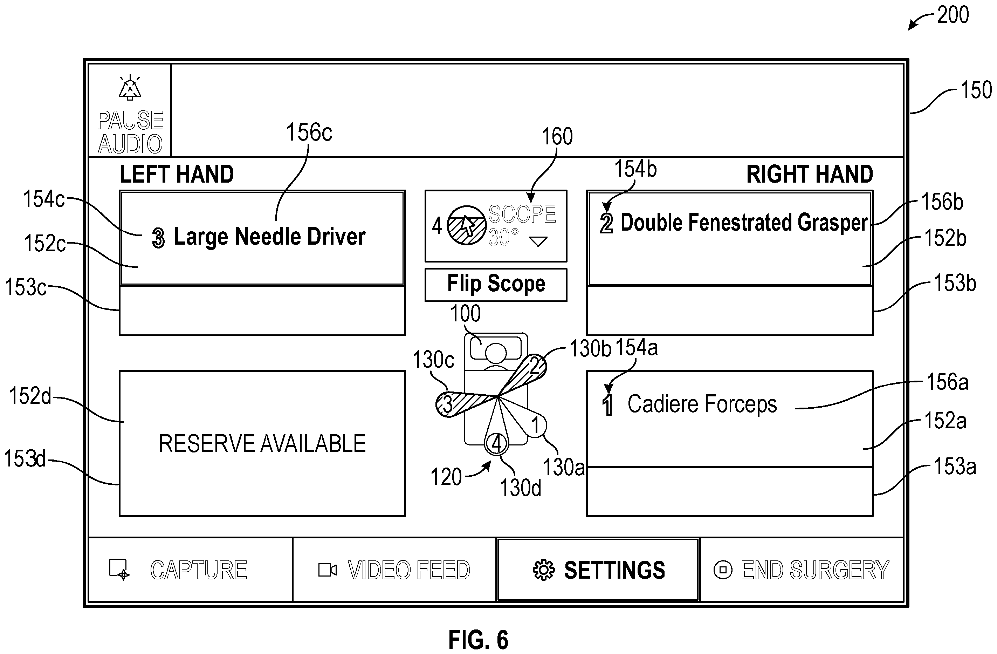

As shown in an exemplary display 200 of FIG. 6. The display 200 may be any of the displays 23, 32, and 34 shows a graphical user interface 150, which includes a plurality of regions 153a-d having graphical representations 152a-c for each of the three robotic arms 40, which are numbered "1"-"3" and a reserve graphical representation 152d. Each of the graphical representations 152a-c includes an identification number 154a-c and an instrument type 156a-c. The user interface 150 also includes an orientation indicator 160. The orientation indicator 160, which shows rotation and pitch indication of the camera 51, which shows rotation and pitch indication of the camera 51 that is coupled to the robotic arm 40d numbered "4".

The controller 21a calculates remaining instrument life for the instrument 50 based on previous use of the instrument 50. Rather than outputting a percentage of total minutes used vs. the total minutes allowed for the instrument 50, which does not provide any context for how many actual uses remain for the instrument 50, the controller 21a provides the user with a predicted number of uses available.

The controller 21a is configured to execute a use prediction algorithm, which may be embodied as software instructions or an application executable by the controller 21a. Using the algorithm, the controller 21a is configured to predict the total number of uses remaining on the instrument 50 by evaluating the average number of minutes of use for the instrument 50. Thus, if the instrument 50 is used for many short uses, the total number of available uses reported to the user will increase. Conversely, if the instrument 50 is used for many long uses, namely, more minutes per use than for short uses, the total number of available uses is reduced.

The use prediction algorithm provides insight into how many additional uses an instrument likely has remaining based on how the instrument has previously been used. The algorithm is based on the concept that a given instrument 50 has a set total allowed use time before the allowed life of the instrument 50 expires. In another embodiment, the remaining usage life of the instrument 50 may be shown as a percentage, which may be calculated based on the total number of minutes used divided by the total number of minutes allowed for the instrument 50, and showing the user that the instrument 50 is expired once a threshold is reached.

If the instrument 50 is used for a number of short uses, the controller 21a displays a larger denominator to the user, indicating that the instrument 50 will last for more discrete uses as compared to the instrument 50 with the same allowed number of minutes that is used in several long uses. The display for the instrument 50 used in several long uses will have a smaller denominator, indicating to the user that the instrument 50 will not be able to be used for as many discrete uses. The total allowed (i.e., maximum) number of uses of the instrument 50 may be adjusted by the user, hospital, or manufacturer.

Furthermore, the use prediction algorithm according to the present disclosure also provides a higher likelihood of the instrument 50 being disabled prior to exceeding the allowed use life. The algorithm utilizes the information gathered about average minutes per use and disables the instrument 50 if the minutes remaining are less than the average minutes per use that the instrument 50 had previously experienced. Disabling the instrument may include any prevention of actuation of the instrument 50, such as stopping the IDU 52 from receiving any user inputs from the surgeon console 30, ignoring user inputs at the surgeon console 30, etc. In addition, disabling the instrument may include preventing an opening/closing of jaws of the instrument, preventing a pivoting or articulation of the jaws or distal end or shaft of the instrument, preventing a rotation of the jaws of the instrument, or any other type of movement of the jaws or shaft of the instrument contemplated by one of skill in the art.

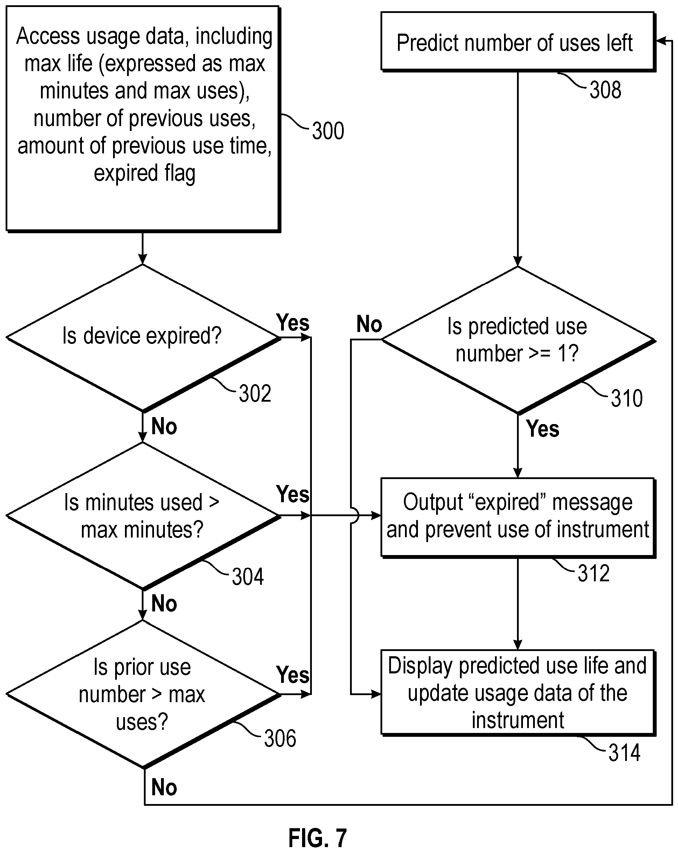

With reference to FIG. 7, for each instrument 50 that is connected to the robotic arm 40, the controller 21a executed the following use prediction algorithm. Initially, at step 300, the usage data from the memory 106 of the instrument 50 is accessed by the controller 21a. Usage data includes instrument's maximum (i.e., allowed) life data, which includes maximum uses and maximum time. Use of the instrument 50 may constitute coupling the instrument 50 and activating the drive couplers 102a-d by the IDU 52. Thus, once this event occurs, the usage count is incremented by the controller 21a. Maximum life values may be preset at the factory for each of the instruments 50.

Usage data may also store previous use data, which includes a number of previous uses, total amount of time of previous uses, and/or an expiration flag, which may be a boolean (i.e., true or false) indicating that the instrument 50 has reached its maximum allowed life.

In embodiments, usage data for each instrument 50 may be stored at another device, such as the control tower 20 and/or a remote server (not shown). Usage data may be stored in a lookup data table and accessed by the controller 21a using an identifier associated with the instrument 50. The identifier may be stored in the memory 106 or any other suitable storage means, such as an RFID or a QR code, and the like.

Once the controller 21a receives the usage data, the controller 21a performs the following verification checks to determine whether the instrument 50 may be used. This includes, at step 302, the controller 21a determining if the expiration flag has been set to "true", which may be set after pervious use of the instrument 50, then the controller 21a outputs an "expired" message 170 as shown in FIG. 6 at step 312. The message may be an audio and/or visual alarm, button, region, window, a scrolling chevron, and/or any other animation shown on the displays 23, 32, and 34. The controller 21a also prevents the surgical robotic system 10 from using the instrument 50. If the expiration flag is "false", the controller 21a proceeds to the next verification.

The next verification, at step 304, includes comparing the total number of prior minutes that the instrument 50 was used for is compared to the maximum allowed time. If the previous use time exceeds the maximum allowed time, then the controller 21a outputs the "expired" message 170 and disables the instrument 50. Otherwise, the controller 21a proceeds to the next verification.

Another verification at step 306 includes comparing the total number of prior uses that the instrument 50 was used for is compared to the maximum allowed number of uses. If the previous use time exceeds the maximum allowed use number, then the controller 21a outputs the "expired" message 170 and disables the instrument 50. Otherwise, the controller 21a proceeds to the next verification. When a new instrument 50 is installed, the controller 21a may output a "new instrument" message 172 stating that this is the first use of the instrument 50.

In addition to the previous verifications, the controller 21a also calculates a predicted number of uses remaining at step 308. The prediction includes calculating an average use time by dividing previous use time by previous number of uses. Thereafter, the process includes dividing the maximum allowed time by the average use time to obtain a predicted number of uses. If the predicted number of uses is less than one, then the controller 21a outputs the "expired" message 170 and disables the instrument 50. Otherwise, the controller 21a outputs a message at step 314 indicating that the instrument 50 may be used and may also output the predicted number of uses remaining as a value.

Predicted use value of the instrument 50 may also be displayed as a ratio 173, as shown in FIG. 6. As described above, the ratio shows "1" as the numerator and the predicted use value as the denominator. The predicted use value may be calculated by dividing the prior use number by the predicted number of uses. In addition, a percentage value and/or a bar graph 174 showing remaining life of the device may be shown. The percentage of life remaining may be calculated based on minutes used divided by the maximum allowed time and converting the ratio into a percentage (e.g., subtracting from 1).

It is envisioned that any of the verifications (i.e., expiration flag, time verification, use verification, predicted use verification) of the use prediction algorithm may be used alone or in any combination, thus, the predicted use verification may be used on its own or in combination with any of the other verifications. Furthermore, the use prediction algorithm may be used for counting surgical instruments or other surgical appliances that are used with surgical systems, such as electrosurgical, ultrasonic, and powered surgical systems.

It will be understood that various modifications may be made to the aspects disclosed herein. In aspects, the color-coded relative position, angular orientation, and operational status of each robotic arm may also be simultaneously viewable on multiple displays. Therefore, the above description should not be construed as limiting, but merely as exemplifications of various aspects. Those skilled in the art will envision other modifications within the scope of the claims appended thereto.

## Claims

1. A surgical robotic system (10) comprising:
a robotic arm (40);
a surgical instrument (50) configured to couple to the robotic arm (40); and
a controller (21a) configured to:
access usage data pertaining to the surgical instrument (50), wherein the usage data includes maximum life data and previous use data,
**characterized in that**:
the maximum life data includes a maximum number of uses and a maximum use time, and the previous use data includes a number of previous uses, a previous use time, and an expired flag; and
the controller (21a) is further configured to:
calculate an average use time for the surgical instrument (50) based on the previous use time and the number of previous uses;
calculate a use time remaining for the surgical instrument (50) based on the previous use time and the maximum use time;
calculate a predicted number of uses remaining for the surgical instrument (50) based on the use time remaining and the average use time; and
disable the surgical instrument (50) if the use time remaining is less than the average use time.

2. The surgical robotic system according to claim 1, wherein the controller (21a) is further configured to disable the surgical instrument (50) based on the expired flag being set to true.

3. The surgical robotic system according to claim 1 or 2, wherein the controller (21a) is further configured to disable the surgical instrument (50) if the previous use time exceeds the maximum use time.

4. The surgical robotic system according to any preceding claim, wherein the controller (21a) is further configured to disable the surgical instrument (50) based on the number of previous uses exceeding the maximum number of uses and disabling includes preventing actuation of the surgical instrument (50) in response to user input.

5. The surgical robotic system according to any preceding claim, further comprising:
a display (23, 32, 34, 200) configured to output a graphical user interface (150) including a usage indicator status pertaining to the surgical instrument (50).

6. The surgical robotic system according to any preceding claim, wherein the surgical instrument (50) includes a storage device (106) configured to store the usage data.

7. The surgical robotic system according to any preceding claim, wherein the controller (21a) is further configured to update the usage data following use of the surgical instrument (50).

8. A computer-implemented method for controlling a surgical robotic system (10) according to any preceding claim, the method comprising:
accessing usage data pertaining to the surgical instrument wherein the usage data includes maximum life data and previous use data;
calculating the predicted number of uses remaining for the surgical instrument (50);
wherein calculating the predicted number of uses remaining for the surgical instrument comprises the following steps: calculate an average use time for the surgical instrument (50) based on the previous use time and the number of previous uses; calculate a use time remaining for the surgical instrument (50) based on the previous use time and the maximum use time; calculate a predicted number of uses remaining for the surgical instrument (50) based on the use time remaining and the average use time;
and
disabling the surgical instrument (50) if the predicted number of uses is less than 1, wherein disabling includes preventing actuation of the surgical instrument in response to user input.

9. The method according to claim 8, further comprising:
displaying on a graphical user interface a usage indicator status pertaining to the surgical instrument.

10. The method according to claim 8, further comprising updating the usage data following use of the surgical instrument.

## Patentansprüche

1. Chirurgisches Robotersystem (10), umfassend:
einen Roboterarm (40);
ein chirurgisches Instrument (50), das zur Kopplung mit dem Roboterarm (40) konfiguriert ist; und
eine Steuerung (21a), die konfiguriert ist zum:
Zugreifen auf Nutzungsdaten bezüglich des chirurgischen Instruments (50), wobei die Nutzungsdaten Daten zur maximalen Lebensdauer und Daten zur vorherigen Nutzung einschließen,
**dadurch gekennzeichnet, dass:**
die Daten zur maximalen Lebensdauer eine maximale Anzahl von Verwendungen und eine maximale Verwendungsdauer einschließen, und die Daten zur vorherigen Verwendung eine Anzahl vorheriger Verwendungen, eine vorherige Verwendungsdauer und eine Ablaufflagge einschließen; und
die Steuerung (21a) ferner konfiguriert ist zum:
Berechnen einer durchschnittlichen Verwendungsdauer für das chirurgische Instrument (50) basierend auf der vorherigen Verwendungsdauer und der Anzahl vorheriger Verwendungen;
Berechnen einer verbleibenden Verwendungsdauer für das chirurgische Instrument (50) basierend auf der vorherigen Verwendungsdauer und der maximalen Verwendungsdauer;
Berechnen einer vorhergesagten Anzahl verbleibender Verwendungen für das chirurgische Instrument (50) basierend auf der verbleibenden Verwendungsdauer und der durchschnittlichen Verwendungsdauer; und
Deaktivieren des chirurgischen Instruments (50), wenn die verbleibende Verwendungsdauer geringer als die durchschnittliche Verwendungsdauer ist.

2. Chirurgisches Robotersystem nach Anspruch 1, wobei die Steuerung (21a) ferner konfiguriert ist, um das chirurgische Instrument (50) zu deaktivieren, basierend darauf, dass die Ablaufflagge auf "wahr" gesetzt ist.

3. Chirurgisches Robotersystem nach Anspruch 1 oder 2, wobei die Steuerung (21a) ferner konfiguriert ist, um das chirurgische Instrument (50) zu deaktivieren, wenn die vorherige Verwendungsdauer die maximale Verwendungsdauer überschreitet.

4. Chirurgisches Robotersystem nach einem der vorstehenden Ansprüche, wobei die Steuerung (21a) ferner konfiguriert ist, um das chirurgische Instrument (50) basierend auf der Anzahl vorheriger Verwendungen, die die maximale Anzahl von Verwendungen überschreitet, zu deaktivieren, und wobei das Deaktivieren das Verhindern der Betätigung des chirurgischen Instruments (50) als Reaktion auf eine Benutzereingabe einschließt.

5. Chirurgisches Robotersystem nach einem der vorstehenden Ansprüche, ferner umfassend:
eine Anzeige (23, 32, 34, 200), die konfiguriert ist, um eine grafische Benutzerschnittstelle (150) auszugeben, die einen Nutzungsanzeigestatus in Bezug auf das chirurgische Instrument (50) einschließt.

6. Chirurgisches Robotersystem nach einem der vorstehenden Ansprüche, wobei das chirurgische Instrument (50) eine Speichervorrichtung (106) einschließt, die zum Speichern der Nutzungsdaten konfiguriert ist.

7. Chirurgisches Robotersystem nach einem der vorstehenden Ansprüche, wobei die Steuerung (21a) ferner konfiguriert ist, um die Nutzungsdaten nach der Verwendung des chirurgischen Instruments (50) zu aktualisieren.

8. Computerimplementiertes Verfahren zum Steuern eines chirurgischen Robotersystems (10) nach einem der vorstehenden Ansprüche, wobei das Verfahren umfasst:
Zugreifen auf Nutzungsdaten bezüglich des chirurgischen Instruments, wobei die Nutzungsdaten Daten zur maximalen Lebensdauer und Daten zur vorherigen Nutzung einschließen;
Berechnen der vorhergesagten Anzahl verbleibender Verwendungen für das chirurgische Instrument (50);
wobei das Berechnen der vorhergesagten Anzahl verbleibender Verwendungen für das chirurgische Instrument die folgenden Schritte umfasst: Berechnen einer durchschnittlichen Verwendungsdauer für das chirurgische Instrument (50) basierend auf der vorherigen Verwendungsdauer und der Anzahl vorheriger Verwendungen; Berechnen einer verbleibenden Verwendungsdauer für das chirurgische Instrument (50) basierend auf der vorherigen Verwendungsdauer und der maximalen Verwendungsdauer; Berechnen einer vorhergesagten Anzahl verbleibender Verwendungen für das chirurgische Instrument (50) basierend auf der verbleibenden Verwendungsdauer und der durchschnittlichen Verwendungsdauer; und
Deaktivieren des chirurgischen Instruments (50), wenn die vorhergesagte Anzahl verbleibender Verwendungen kleiner als 1 ist, wobei das Deaktivieren das Verhindern der Betätigung des chirurgischen Instruments als Reaktion auf eine Benutzereingabe einschließt.

9. Verfahren nach Anspruch 8, ferner umfassend:
Anzeigen eines Nutzungsindikatorstatus bezüglich des chirurgischen Instruments auf einer grafischen Benutzerschnittstelle.

10. Verfahren nach Anspruch 8, ferner umfassend das Aktualisieren der Nutzungsdaten nach der Verwendung des chirurgischen Instruments.

## Revendications

1. Système robotique chirurgical (10) comprenant :
un bras robotique (40) ;
un instrument chirurgical (50) conçu pour s'accoupler au bras robotique (40) ; et
un dispositif de commande (21a) configuré pour :
accéder aux données d'utilisation relatives à l'instrument chirurgical (50), dans lequel les données d'utilisation comportent les données de durée de vie maximale et les données d'utilisation précédente,
**caractérisé en ce que** :
les données de durée de vie maximale comportent un nombre maximal d'utilisations et une durée maximale d'utilisation, et les données d'utilisation précédentes comportent un nombre d'utilisations précédentes, une durée d'utilisation précédente et un indicateur de péremption ; et
le dispositif de commande (21a) est en outre configuré pour :
calculer une durée d'utilisation moyenne de l'instrument chirurgical (50) en fonction de la durée d'utilisation précédente et du nombre d'utilisations précédentes ;
calculer une durée d'utilisation restante de l'instrument chirurgical (50) en fonction de la durée d'utilisation précédente et de la durée d'utilisation maximale ;
calculer un nombre prédit d'utilisations restantes de l'instrument chirurgical (50) en fonction du temps d'utilisation restant et du temps d'utilisation moyen ; et
désactiver l'instrument chirurgical (50) si le temps d'utilisation restant est inférieur au temps d'utilisation moyen.

2. Système robotique chirurgical selon la revendication 1, dans lequel le dispositif de commande (21a) est en outre configuré pour désactiver l'instrument chirurgical (50) en fonction de l'indicateur de péremption qui est réglé sur vrai.

3. Système robotique chirurgical selon la revendication 1 ou 2, dans lequel le dispositif de commande (21a) est en outre configuré pour désactiver l'instrument chirurgical (50) si la durée d'utilisation précédente dépasse la durée d'utilisation maximale.

4. Système robotique chirurgical selon l'une quelconque revendication précédente, dans lequel le dispositif de commande (21a) est en outre configuré pour désactiver l'instrument chirurgical (50) en fonction du nombre d'utilisations précédentes dépassant le nombre maximal d'utilisations, et la désactivation comporte l'empêchement de l'actionnement de l'instrument chirurgical (50) en réponse à l'entrée de l'utilisateur.

5. Système robotique chirurgical selon l'une quelconque revendication précédente, comprenant en outre :
un affichage (23, 32, 34, 200) configuré pour produire une interface utilisateur graphique (150) comportant un état d'indicateur d'utilisation relatif à l'instrument chirurgical (50).

6. Système robotique chirurgical selon l'une quelconque revendication précédente, dans lequel l'instrument chirurgical (50) comporte un dispositif de stockage (106) configuré pour stocker les données d'utilisation.

7. Système robotique chirurgical selon l'une quelconque revendication précédente, dans lequel le dispositif de commande (21a) est en outre configuré pour mettre à jour les données d'utilisation après l'utilisation de l'instrument chirurgical (50).

8. Procédé mis en œuvre par ordinateur permettant de contrôler un système robotique chirurgical (10) selon l'une quelconque revendication précédente, le procédé comprenant :
l'accès aux données d'utilisation relatives à l'instrument chirurgical, dans lequel les données d'utilisation comportent les données de durée de vie maximale et les données d'utilisation précédentes ;
le calcul du nombre prédit d'utilisations restantes pour l'instrument chirurgical (50) ;
dans lequel le calcul du nombre prédit d'utilisations restantes de l'instrument chirurgical comprend les étapes suivantes : le calcul d'une durée d'utilisation moyenne pour l'instrument chirurgical (50) en fonction de la durée d'utilisation précédente et du nombre d'utilisations précédentes ; le calcul d'une durée d'utilisation restante de l'instrument chirurgical (50) en fonction de la durée d'utilisation précédente et de la durée d'utilisation maximale ; le calcul d'un nombre prédit d'utilisations restantes de l'instrument chirurgical (50) en fonction du temps d'utilisation restant et du temps d'utilisation moyen ; et
la désactivation de l'instrument chirurgical (50) si le nombre prédit d'utilisations est inférieur à 1, dans lequel la désactivation comporte l'empêchement de l'actionnement de l'instrument chirurgical en réponse à l'entrée de l'utilisateur.

9. Procédé selon la revendication 8, comprenant en outre :
l'affichage sur une interface utilisateur graphique d'un état d'indicateur d'utilisation relatif à l'instrument chirurgical.

10. Procédé selon la revendication 8, comprenant en outre la mise à jour des données d'utilisation après l'utilisation de l'instrument chirurgical.
